Europäisches Patentamt

(19) **European Patent Office**

**Office européen des brevets**

(11) Publication number: **0 229 485**
**A2**

# (12) EUROPEAN PATENT APPLICATION

(21) Application number: **86309416.5**

(22) Date of filing: **03.12.86**

(51) Int. Cl.³: **C 12 N 5/00**

(30) Priority: **09.01.86 DE 3600429**
**18.06.86 US 875896**
**14.10.86 US 918654**

(43) Date of publication of application:
**22.07.87 Bulletin 87/30**

(84) Designated Contracting States:
**AT BE CH DE ES FR GB GR IT LI LU NL SE**

(71) Applicant: **SPECTRAL BIOANALYSIS LTD.**
**12 York Gate**
**London NW1 4QS(GB)**

(72) Inventor: **Kübler, Ulrich, Dr.**
**28 Knoebelstrasse**
**D-8000 Munich 22(DE)**

(74) Representative: **Woodcraft, David Charles et al,**
**BROOKES & MARTIN High Holborn House 52/54 High Holborn**
**London, WC1V 6SE(GB)**

(54) **Growth-promoting cell culture medium and process for the cultivation of cells employing the same.**

(57) A growth-promoting cell culture medium comprising a basic cell culture medium, and additionally, a bivalent zinc compound in an amount sufficient to promote cell growth and a process for cultivating cells employing the growth-promoting cell culture medium.

EP 0 229 485 A2

Croydon Printing Company Ltd.

# GROWTH-PROMOTING CELL CULTURE MEDIUM AND PROCESS FOR THE CULTIVATION OF CELLS EMPLOYING THE SAME

## FIELD OF THE INVENTION

The present invention relates to a growth-promoting cell culture medium comprising a basic cell culture medium, and additionally, bivalent zinc in an amount sufficient to promote cell growth. The present invention also relates to a process for cultivating cells employing the growth-promoting cell culture medium.

## BACKGROUND OF THE INVENTION

Cell culture media which are conventionally employed in the art contain inorganic and organic components. Conventional cell culture media, although allowing some cell growth, are disadvantageous since such do not give rise to satisfactory stable growth of cells or to satisfactory cell and cellular protein yields, i.e., do not promote cell growth.

## SUMMARY OF THE INVENTION

An object of the present invention is to provide a cell culture medium which allows for stable growth of cells.

Another object of the present invention is to provide a culture medium which allows for high cell and cellular protein yields.

An even further object of this invention is to provide a culture medium particularly useful for the growth of eukaryotic, e.g., mammalian, cells to achieve high cell growth and cellular protein yields.

Still another object of the present invention is to provide a method for cultivating cells such that the growth thereof is promoted.

The above-described objects of the present invention have been met by the cell growth medium of this invention comprising a basic cell culture medium and bivalent zinc, wherein the bivalent zinc is present in a concentration of 0.8 to 1.6 mg of $Zn^{++}$/l of the culture medium.

## BRIEF DESCRIPTION OF THE DRAWINGS

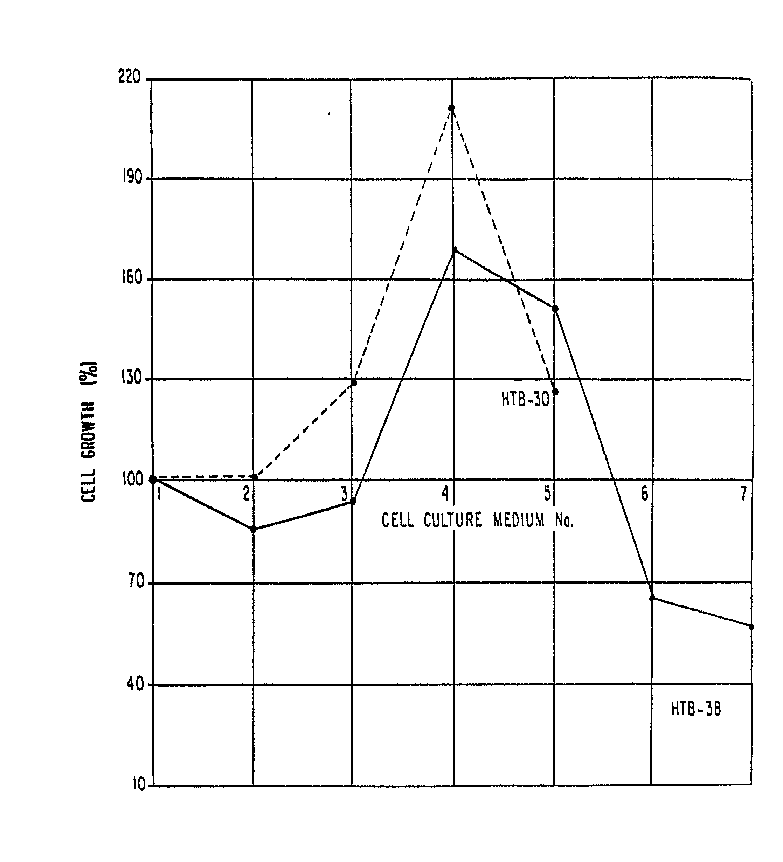

The Figure shows the cell growth, expressed by the protein concentration, for the transformed cell lines HTB-38 and HTB-30.

## DETAILED DESCRIPTION OF THE INVENTION

The basic cell culture medium of the present invention can be any conventional basic cell culture medium containing a source of carbon, a source of nitrogen and other inorganic and organic components such as vitamins, amino acids and the like, e.g., as disclosed in H.J. Morton, In Vitro 6:89-108 (1970), the disclosure of which is incorporated by reference. The basic cell culture medium employed in the process of the present invention will depend upon the type of cells being cultured, i.e. mammalian, reptilian, avian or insect, each of which is well known to ones skilled in the art, has different growth requirements. Further, the basic cell culture medium of the present invention is one which can be employed to culture either transformed cells or non-transformed rapidly dividing cells of mammalian, reptilian, insect or avian origin. Commercially available examples of such basic cell culture mediums, e.g., for mammalian cells, to which the present invention is applicable include HAM F-10, HAM F-12 Eagle's Minimum Essential Medium (MEM), Puck's N-15 and Puck's N-16, McCoy 5A (mod.), RPMI 1603, 16344, 1640, and the like. The preferred basic cell culture medium employed in the present invention is McCoy 5A (mod.).

As defined herein, the term "non-transformed" rapidly dividing cells includes cells of embryonic and epithelial origin and the term "transformed" cells includes cells of malignant or hybridomal origin (human or non-human).

Examples of such transformed cells include embryonal carcinoma, testis, metastasis to lymph node Cales-1 B (ATCC No. HTB-104), breast HBL-100 (ATCC No. HTB-124), endometrial adenocarcinoma HEC-1-A (ATCC No. HTB-112) Walker rat carcinoma LLC-URC 256 (ATCC No. CCL-38), HT-29 (ATCC No. HTB-38) and SK-BR-3 (ATCC No. HTB-30). The present invention can in particular be used with transformed mammalian cells such as malignant and hybridoma cells.

Examples of such non-transfomred cells include human lung diploid MRC-5 (ATCC No. CCL-171). WI-38 (ATCC No. CCL-75) and IMR-90 (ATCC No. CCL-186).

Basic cell culture media which are conventionally employed in the art contain inorganic and organic components along with trace amounts of bivalent zinc in the form of e.g., $ZnSO_4$. The concentration of the bivalent zinc in such basic culture medium, e.g., HAM F-10 and HAM F-12 (Serva Company) is about 0.007 to 0.2 mg of $Zn^{++}$/l of the culture medium. However, cell culture media containing such trace amounts of bivalent zinc do not give rise to a growth promoting effect. It has been found in the present invention that when the amount of bivalent zinc in the culture medium is above trace amounts and within the above-described range, a growth promoting effect is achieved.

The basic cell culture medium according to the present invention contains a bivalent zinc in an amount sufficient to promote growth, e.g., about 0.8 to about 1.6 mg of $Zn^{++}/l$, preferably 1.0 to 1.4 mg of $Zn^{++}/l$, in the culture medium. The most preferred concentration of the bivalent zinc is around 1.2 mg of $Zn^{++}/l$ since the maximum cell yield is obtained using such a concentration.

The bivalent zinc used in the present invention can be from any zinc source and the source is not limiting. Suitable sources of the bivalent zinc include any non-toxic inorganic bivalent zinc compound or non-toxic organic bivalent zinc compound. Examples of such non-toxic inorganic bivalent zinc compounds include $ZnCl_2$, ZnO, $ZnCO_3$ and $ZnSO_4$. The preferred non-toxic inorganic bivalent zinc compound which can be employed in the present invention is $ZnCl_2$. Examples of such non-toxic organic bivalent zinc compounds which can be employed in the present invention include zinc salts of organic compounds such as zinc acetate. Mixtures of · the above-described bivalent zinc compounds can also be employed in the present invention if desired.

The bivalent zinc can be incorporated into the basic cell culture medium using any method, for example, the above-described bivalent zinc compounds can be added ·to the basic cell culture medium in solid form, although it is preferable to add such in the form of a solution such as in an aqueous solution, in an aqueous acidic solution or in an aqueous buffered solution.

The high cell and cellular protein yield obtained using the cell culture medium of the present invention has considerable advantages in analytical experimentations, e.g., in the processing of cytoplasmic proteins. This applies especially to the accelerated growth of hybridomas and their final products, e.g. antibodies, which can be cultivated quickly and efficiently without the need for cell subcultures or medium changes.

The cells employed in the present invention are cultured in the cell culture medium of the present invention at the optimum known culture conditions for the particular cell, typically at 36.5°C ± 0.5°C, 5% $CO_2$/95% air.

The following non-limiting examples of the present invention are provided for illustrative purposes only and are in no way intended to limit the scope of the present invention. Unless otherwise indicated herein, all parts, percents, ratios and the like are by weight.

## EXAMPLE

In this example, malignant transformed human cell lines obtained from the American Type Culture Collection, i.e., a human adenocarcinoma carcinoma cell line of the colon, HT-29 (ATCC No. HTB-38) and the human adenocarcinoma carcinoma cell line of the breast, SK-BR-3 (ATCC No. HTB-30) were used. Non-transformed human fibroblast cells (E5, Institute of Applied Cell Culture, Ltd.) were employed as a control. The cell lines were obtained from the American Type Culture Collection in a frozen condition. In order to reactivate the cells, the cells were subcultured in McCoy 5A (mod.) medium (Serva Company) containing 15% (v/v) fetal calf serum.

The reactivated cells were allowed to reach the logarithmic growth phase and then detrypsinized and harvested. The harvested cells were washed twice with McCoy 5A (mod.) medium containing 15% (v/v) fetal calf serum and then resuspended in the same medium at a concentration of $10^6$ cells per ml. The correct cell concentration was determined using a Neubeuer-Turk count chamber in which the viability of the cells was also determined by means of Trypan blue coloring (see: Freshney, I.R., *Culture of Animal Cells*, Alan Riss Publ. Co., New York, N.Y. (1983)). The viability of the HTB-28 cells was 95% and that of the HTB-30 cells was 78%. The viability of the human fibroblast cells (E5) was 95%.

To tubes containing 200,000 HTB-30 cells or 250,000 HTB-38 cells was added Cell Culture Medium Nos. 1, 2, 3, 4 or 5 described below and the cells were incubated for 80 hours or added Cell Culture Medium Nos. 6 and 7 described below and the cells were incubated for 96 hours. In addition, to tubes containing 200,000 human fibroblast cells (E5) were added Cell Culture Medium Nos. 1 and 4 described below and the cells were incubated for 72 hours. All of the cells were incubated at 36.5°C (± 0.5°C), 5% $CO_2$ and 95% air. Five duplicate tubes were employed for each cell culture medium for each cell.

Cell Culture Medium No. 1 comprised McCoy 5A (mod.) medium plus 15% (v/v) fetal calf serum without the addition of bivalent zinc. McCoy 5A (mod.) medium only contains trace amounts of bivalent zinc, i.e., 0.0 ± 0.001 mg of $Zn^{++}$/1.

Cell Culture Medium No. 2 was identical to Cell Culture Medium No. 1 except that $ZnCl_2$ was added to a final concentration of 0.2 mg of $Zn^{++}$/1.

Cell Culture Medium No. 3 was identical to Cell Culture Medium No. 1 except that $ZnCl_2$ was added to a final concentration of 0.8 mg of $Zn^{++}$/1.

Cell Culture Medium No. 4 was identical to Cell Culture Medium No. 1 except that $ZnCl_2$ was added to a final concentration of 1.2 mg of $Zn^{++}$/1.

Cell Culture Medium No. 5 was identical to Cell Culture Medium No. 1 except that $ZnCl_2$ was added to a final concentration of 1.6 mg of $Zn^{++}$/1.

Cell Culture Medium No. 6 was identical to Cell Culture Medium No. 1 except that $ZnCl_2$ was added to a final concentration of 2.1 mg of $Zn^{++}/l$.

Cell Culture Medium No. 7 was identical to Cell Culture Medium No. 1 except that $ZnCl_2$ was added to a final concentration of 2.8 mg of $Zn^{++}/l$.

After the end of the incubation time, the culture medium was removed and the cells were washed three times with Dulbecco buffer solution (see: Dulbecco, R.U., Vogt, M., _J. Exp. Med._ 98:167-182 (1954)). Next, the cells were dried and octyl phenol polyethylene glycol ether was added at a concentration of 0.2% (v/v) to each tube to which 20 µl of 1.0 N NaOH had been additionally added per ml.

After thorough mixing and allowing the samples to stand for 30 minutes at 20-25°C, 200 µl each of the resulting cell extracts were used for protein determinations according to the Lowry method (see: Lowry, O.H. et al., _J. Biol. Chem._ 193:265 (1951)).

The results obtained are shown in the Table below. The Control in the Table below refers to Cell Culture Medium No. 1 at 4°C to which no cells had been added. In the Table, the growth percentage is determined based upon Cell Culture Medium No. 1, which does not contain bivalent zinc above trace amounts, giving rise to a 100% growth value. All growth values are mean values from the five duplicate tubes ± the standard deviation.

The growth value as a percent of the Control was determined according to the following formula:

$$\% \text{ growth} = 100 \times \frac{\text{Absorption of Sample} - \text{Absorption of Control (initial value)}}{\text{Absorption of Sample in Medium No. 1} - \text{Absorption of Control (initial value)}}$$

0229485

In addition, in the Table, the mean adsorption values are determinative of the concentration of protein in the cells and were evaluated at 660 nm.

The mean absorption values for two concentrations of bovine serum albumin (BSA) were measured as shown below and used a control for the protein concentration determinations:

(a)  BSA 100 µg/tube:    0.270   0.280   0.248   0.265
(b)  BSA  50 µg/tube:    0.135   0.146   0.143   0.125

| Samples | HTB-38 | | HTB-30 | | Human Fibroblasts | |
|---|---|---|---|---|---|---|
| | Mean adsorption values | % growth | Mean adsorption values | % growth | Mean adsorption Values | % growth |
| Control | 0.206 ± 0.032 | --- | 0.198 ± 0.023 | --- | 0.179 ± 0.027 | --- |
| Medium No. 1 | 0.935 ± 0.076 | 100 | 0.699 ± 0.033 | 100 | 0.519 ± 0.012 | 100 |
| Medium No. 2 | 0.830 ± 0.025 | 85.6 ± 3.0 | 0.700 ± 0.036 | 100.2 ± 5.14 | --- | --- |
| Medium No. 3 | 0.890 ± 0.086 | 93.8 ± 9.7 | 0.844 ± 0.037 | 128.9 ± 4.4 | --- | --- |
| Medium No. 4 | 1.430 ± 0.125 | 167.9 ± 8.9 | 1.128 ± 0.262 | 211.2 ± 19.6 | 0.543 ± 0.025 | 107 |
| Medium No. 5 | 1.308 ± 0.056 | 151.2 ± 4.3 | 0.831 ± 0.084 | 126.3 ± 10.1 | --- | --- |
| Medium No. 6* | 0.882 ± 0.136 | 65.3 ± 15.4 | --- | --- | --- | --- |
| Medium No. 7* | 0.799 ± 0.057 | 57.2 ± 7.13 | --- | --- | --- | --- |

* For the assays with Medium Nos. 6 and 7, the mean absorption value for the Control was 0.221 ± 0.046 and the mean absorption value for Medium No. 1 was 1.232 ± 0.098.

The growth values shown in the Table above for HTB-38 and HTB-30 cells are graphically illustrated in the Figure. The Figure shows that the growth rate, expressed as protein concentration, is promoted for HTB-38 cells and HTB-30 cells when grown in a basic cell culture medium containing from about 0.8 to about 1.6 mg of $Zn^{++}$/l of bivalent zinc.

Specifically, the Table and the Figure clearly demonstrate that both cell lines HTB-38 and HTB-30 exhibit a 28.9% increase in protein yield when bivalent zinc is employed in the basic culture medium in an amount of about 0.8 and about 1.0 mg of $Zn^{++}$/l. In addition, the results in the Table demonstrate that a maximum growth is achieved when bivalent zinc is employed in an amount 1.0 mg of $Zn^{++}$/l of the basic cell culture medium, i.e., cell line HTB-38 had a 67.9% greater yield than Cell Culture Medium No. 1 and cell line HTB-30 had an 111.2% better yield than Cell Culture Medium No. 1.

The values in the Table and the Figure also demonstrate that when bivalent zinc is added in a final concentration of 0.2 mg of $Zn^{++}$/l of medium, i.e., a level higher than the trace amount of bivalent zinc normally found in basic cell culture media, or 2.1 mg of $Zn^{++}$/l or higher, no growth increasing effect was seen and in fact a growth decreasing effect was seen with HTB-38.

The Table also clearly demonstrates the growth of non-transformed cells non-rapidly dividing cells, i.e., human fibroblasts (E5) is not sufficiently promoted when grown in basic cell culture medium containing bivalent zinc above trace amounts and thus that bivalent zinc is unexpectedly necessary for the promotion of growth of rapidly dividing cells.

While the present invention has been described in detail and with respect to specific embodiments thereof, it would apparent that changes and modifications can be made therein without departing from the
spirit and scope thereof.

**0229485**

CLAIMS

1.    A cell culture medium comprising a basic cell culture medium and additionally bivalent zinc in an amount sufficient to promote cell growth.

2.    The cell culture medium as claimed in Claim 1, wherein said bivalent zinc is present in an amount of from about 0.8 to about 1.6 mg of $Zn^{++}$/l.

3.    The cell culture medium as claimed in Claim 2, wherein said bivalent zinc is present in an amount of from about 1.0 to 1.4 mg of $Zn^{++}$/l.

4.    The cell culture medium as claimed in Claim 3, wherein said bivalent zinc is present in an amount of about 1.2 mg of $Zn^{++}$/l.

5.    The cell culture medium as claimed in Claim 1, wherein said basic cell culture medium comprises a cell culture medium for the growth of transformed cells or non-transformed rapidly dividing cells.

6.    The cell culture medium as claimed in Claim 5, wherein said basic cell culture medium is a cell culture medium for the growth of transformed mammalian cells.

7.    The cell culture medium as claimed in Claim 5, wherein said basic cell culture medium comprises a cell culture medium for the growth of hybridoma cells.

8.    The cell culture medium as claimed in Claim 1, wherein the source of said bivalent zinc is a compound selected from the group consisting of non-toxic organic bivalent zinc compounds and non-toxic inorganic bivalent zinc compounds.

9.    The cell culture medium as claimed in Claim 8, wherein said non-toxic organic bivalent zinc compounds are zinc salts of organic compounds.

10. The cell culture medium as claimed in Claim 8, wherein said non-toxic inorganic bivalent zinc compounds are selected from the group consisting of $ZnCl_2$, $ZnO$, $ZnCO_3$ and $ZnSO_4$.

11. The cell culture medium as claimed in Claim 10, wherein said non-toxic inorganic bivalent zinc compound is $ZnCl_2$.

12. A process for culturing cells comprising culturing cells in the presence of a cell culture medium comprising a basic cell culture medium and additionally bivalent zinc in an amount sufficient to promote cell growth.

13. The process for culturing cells as claimed in Claim 12, wherein said bivalent zinc is present in an amount of from about 0.8 to about 1.6 mg of $Zn^{++}/l$.

14. The process for culturing cells as claimed in Claim 13, wherein said bivalent zinc is present in an amount of from 1.0 to 1.4 mg of $Zn^{++}/l$.

15. The process for culturing cells as claimed in Claim 14, wherein said bivalent zinc is present in an amount of about 1.2 mg of $Zn^{++}/l$.

16. The process for culturing cells as claimed in Claim 12, wherein said basic cell culture medium comprises a cell culture medium for the growth of transformed cells or non-transformed rapidly dividing cells.

17. The process for culturing cells as claimed in Claim 16, wherein said basic cell culture medium is a cell culture medium for the growth of transformed mammalian cells.

18. The process for culturing cells as claimed in Claim 16, wherein said basic cell culture medium comprises a cell culture medium for the growth of hybridoma cells.

19. The process for culturing cells as claimed in Claim 12, wherein the source of said bivalent zinc is a compound selected from the group consisting of non-toxic organic bivalent zinc compounds and non-toxic inorganic bivalent zinc compounds.

20. The process for culturing cells as claimed in Claim 19, wherein said non-toxic organic bivalent compounds are zinc salts of organic compounds.

21. The process for culturing cells as claimed in Claim 19, wherein said non-toxic inorganic bivalent zinc compounds are selected from the group consisting of $ZnCl_2$, ZnO, $ZnCO_3$ and $ZnSO_4$.

22. The process for culturing cells as claimed in Claim 21, wherein said non-toxic inorganic bivalent zinc compound is $ZnCl_2$.